# EUROPEAN PATENT APPLICATION

(11) **EP 4 825 176 A1**
(43) Date of publication of application: **21.10.2026**
(21) Application number: 23961022.3
(22) Date of filing: 12.12.2023
(51) Int. Cl.: A01N 63/20, A01N 63/30, C12N 1/20, C12N 1/14, A01N 25/24, A01N 25/26

(54) **COMPOSITION FOR INCREASING THE STABILITY AND VIABILITY OF AGRICULTURALLY IMPORTANT MICROORGANISMS IN THE COATING OF SEEDS FOR STORAGE; METHOD OF PREPARATION AND USE THEREOF**

(71) Applicant: TOTAL BIOTECNOLOGIA INDÚSTRIA E COMÉRCIO LTDA, 81460-020 Curitiba - PR (BR)
(72) Inventor: POLAQUINI, Carlos Roberto, 13914-552 Jaguariúna - SP (BR); VENDRAMIN BERTOTI, Flávio André, 13912-428 Jaguariúna - SP (BR); MARCOLINO GOMES, Juliana, 803201-00 Curitiba - PR (BR); GOMES, Douglas Fabiano, 803201-00 Curitiba - PR (BR); HIPÓLITO DE ASSIS FILHO, Jonas, 81460-020 Curitiba - PR (BR)
(74) Representative: Vossius & Partner Patentanwälte Rechtsanwälte mbB
(86) International application number: PCT/BR2023/050446
(87) International publication number: WO 2025/123100

(57) **Abstract**

The present invention provides a composition for coating crop seeds with agriculturally important microorganisms. A composition according to the present invention provides stabilization and viability of agriculturally important microorganisms in the coating of stored seeds, resulting in a long shelf-life. The present invention further provides a method for preparing a composition for coating seeds with viable microorganisms, as well as a method for coating seeds with a composition according to the present invention. The agriculturally important microorganisms according to the present invention play multiple biological roles, including, but not limited to, promotion of plant growth, mobilization of nutrients, and the control of plant-parasitic nematodes and phytopathogens.

## Description

### TECHNICAL FIELD OF THE INVENTION

The present invention relates to the field of agriculture, more specifically to the coating of agricultural seeds for the purpose of improving traits of crops grown from coated seeds. The present invention provides a composition for coating seeds of agriculturally important plants, combining functionalities desirable in the field of seed treatment and microorganisms, while preserving their viability until planting. A composition according to the present invention provides stabilization and viability of agriculturally important microorganisms in the coating of stored seeds, resulting in a long shelf-life. Furthermore, the present invention further provides a method for preparing a composition for coating seeds with viable microorganisms. The microorganisms according to the present invention possess multiple biological functions, including, but not limited to, promotion of plant growth, nutrient mobilization, and the control of plant-parasitic nematodes and phytopathogens.

### BACKGROUND OF THE INVENTION

It is widely recognized that most of the crops intended for food production face the risk of being affected by pests and diseases. To meet the constantly increasing demand for food, farmers have widely resorted to the excessive use of synthetic fertilizers and pesticides to mitigate these problems. Although chemical pesticides have contributed to an increased crop production in many countries, they have also caused problems, including decreased soil fertility and degradation of local ecosystems due to soil, water, and air pollution (CHATTERJEE et al., 2017). Therefore, to render agriculture more sustainable, it is essential to expand the adoption of biological alternatives, which are known to promote benefits in both yield and plant health (FERREIRA et al., 2023).

The vast majority of disease-causing agents can be disseminated through seeds, which are highly efficient at spreading pathogens due to their intrinsic characteristics (PARISI and MEDINA, 2013). For this reason, seed treatment is a technique frequently employed by Brazilian farmers and plays a very important role in protecting them from the detrimental action of pests and pathogens, especially during the early stage of seedling development (LUDWIG et al., 2015).

Seed treatment, in a broad sense, comprises the application of processes and substances designed to preserve or improve seed performance, thus allowing the maximum expression of the genetic potential of agricultural crops (MACHADO, 2000). This includes the application of pesticides (fungicides, insecticides and nematicides), biological products, stimulants, micronutrients or physical treatments such as thermotherapy (HENNING, 2012).

In Brazil, the seed treatment market reached USD 654 million in 2020, including insecticides, fungicides, and nematicides. Approximately 90% of Brazilian soybean seeds are treated with fungicides and 80% with insecticides, and in hybrid maize, 100% of the seeds are treated with fungicides and 85% with insecticides (GOULART and NUNES, 2021).

Currently, soybean seed treatment with fungicides is carried out in two ways: *on farm,* which is carried out by the farmer himself on his own farm, and industrial seed treatment (IST), which is the process carried out in seed treatment centers. IST provides advantages such as superior quality due to the precise dosing, coverage, and adhesion of the applied products, in addition to enabling the application of film-coating products based on synthetic polymers, which protect the seeds and enhance their performance in the field (MENTEN and MORAES, 2010).

In this context, incorporating biological control into industrial seed treatment is highly interesting, as it would provide a sustainable alternative to a management practice that is already routinely used by farmers. The main advantage of biological products lies in the possibility of exploring different modes of action, such as antagonism and parasitism in relation to chemical pesticides (ZUCCHI et al., 2008).

However, there are currently no technologies available to enable the use of biological products in association with industrial seed coating, due to challenges inherent to the biology of microorganisms, which often preclude the use of some techniques (BIRD, 2003). Therefore, the development of formulation strategies opens up possibilities for improving the quality and preservation of strains, as well as proposing new ways of applying biological products in the field (FLORENCIO et al., 2022). The formulation step and the association of microorganisms with a suitable carrier and additives, has emerged as an alternative approach for improving shelf life and the effectiveness of biocontrol agents, among other characteristics (SAMMAURIA et al., 2020). Formulations should be designed to ensure the long-term viability of microorganisms during production, storage, transportation, application, and at the inoculation site, thereby providing a stable environment under a wide range of environmental conditions (BASHAN et al., 2014). For these reasons, formulation is a key factor in the success of a bioproduct and can affect successful commercialization. A formulation effectiveness can be demonstrated by the significant increase in parameters such as: fresh and dry biomass, changes in morphology of the plant roots and shoots, increased plant nutrition, yield, and productivity (SOUMARE et al., 2020).

The main challenges in developing an efficient formulation are: increasing the shelf life of the antimicrobial agent, finding a suitable carrier material and additive, and overcoming issues in transportation, storage and application (HERRMANN et al., 2013).

Therefore, there is still a need for a biological formulation that exhibits (i) long survival of agriculturally important microorganisms in the formulation stored at room temperature, (ii) long survival of agriculturally useful microorganisms on seeds treated in conjunction with chemical treatments, and (iii) efficiency at economically viable doses for application in major crops of agricultural relevance.

### BRIEF DESCRIPTION OF THE INVENTION

The present invention provides a seed-coating composition comprising microorganisms that play agriculturally important roles, providing stabilization and viability of the microorganisms present in the coating of stored seeds, resulting in an extended shelf life. The present invention further provides a method for preparing a composition for coating seeds with viable microorganisms. The microorganisms according to the present invention play multiple biological roles, including, but not limited to, promotion of plant growth, mobilization of nutrients, and the control of plant-parasitic nematodes and phytopathogens. Accordingly, the compositions of the present invention enable the preparation of seeds coated with microorganisms that provide desirable agricultural functions while maintaining the viability of such microorganisms for extended periods of time.

The present invention further provides methods for preparing a seed coating composition according to the present invention and for producing coated seeds, as well as a seed coated with a composition according to the present invention.

Additional embodiments and aspects of the present invention will become apparent from the detailed description that follows, the accompanying drawings, and the appended set of claims.

### BRIEF DESCRIPTION OF THE FIGURES

Figure 1 (Fig. 1) depicts the visual appearance of the formulation stored at different temperatures. A = Freshly formulated, B = 14 days at 5 °C, C = 14 days at 28 °C, and D = 14 days at 54 °C.
Figure 2 (Fig. 2) depicts the count of colony-forming units present in the formulation stored at different temperatures.
Storage for 14 days at 54°C mimics two years of storage at room temperature.
Figure 3 (Fig. 3) shows the visual appearance/coating of soybean seeds treated with the formulation of the present invention. The filter paper shows the high adhesion of pigment after shaking the seeds in water.
Figure 4 (Fig. 4) shows bacterial cell recovery counts on soybean seeds treated under different conditions. The seeds were treated and stored at room temperature. The counts were carried out every 30 days over a period of 360 days.
Figure 5 (Fig. 5) shows the visual appearance of soybean seeds treated with different pigments at a dose of 2.5 mL of formulation per kilogram of seed.
Figure 6 (Fig. 6) shows the plant development at 21 days post-emergence. A = control, B = chemical treatment, C = biological formulation, D = chemical treatment associated with the biological formulation.

### DETAILED DESCRIPTION OF THE INVENTION

The present invention provides a seed-coating composition comprising viable microorganisms that play agriculturally important roles, providing stabilization and viability of the microorganisms present in the coating of stored seeds, resulting in an extended shelf life.

A composition according to the present invention comprises:
at least one antifreeze agent in an amount ranging from about 1% to about 20% (w/w);
at least one thickener agent in an amount ranging from about 0.1% to about 5% (w/w);
water in an amount ranging from about 5 to about 20% (w/w);
at least one antifoaming agent in an amount ranging from about 0.1 to about 10% (w/w);
at least one dispersing agent in an amount ranging from about 5 to about 20% (w/w);
at least one biocide in an amount ranging from 0.1 to 10% (w/w);
at least one agriculturally important microorganism in an amount ranging from about 10 to about 60% (w/w); and
at least one adhesive in an amount ranging from about 5 to about 20% (w/w).

Preferably, a composition according to the present invention further comprises a colorant in an amount ranging from about 5 to about 30% (w/w).

In preferred embodiments of the present invention, the antifreeze agent may be, without limitation, propylene glycol, glycerol, hexylene glycol, monoethylene glycol, or diethylene glycol; the thickener may be, without limitation, xanthan gum, guar gum, gum arabic, or carboxymethylcellulose; the antifoaming agent is emulsified silicone; the dispersing agent may be, without limitation, tristyrylphenol phosphate ether, sodium N-methyl oleoyl taurate, styrene-acrylic polymer, sulfosuccinate, tristyrylphenol ethoxylate and tristyrylphenol ethoxylate sulfate, or polysorbate; the biocide may be, without limitation, 1,2-benzisothiazol-3(2H)-one, potassium sorbate, methyl paraben, sodium benzoate, or benzyl alcohol; and the adhesive may be, without limitation, urethane methacrylate, methacrylate ester, acrylic epoxy, or acrylate.

In preferred embodiments of the present invention, the agriculturally important microorganism may be of different genera selected from, but not limited to, the group of species *Bacillus subtilis, Bacillus amyloliquefaciens, Bacillus velezensis, Bacillus pumilus, Bacillus thuringiensis, Bacillus circulans, Bacillus haynesii, Bacillus aryabhattai, Bacillus licheniformis, Paenibacillus durus, Brevibacillus laterosporus, Pseudomonas fluorences, Pseudomoas chlororaphis, Pseudomonas protegens, Azospirillum brasilense, Bradyrhizobium japonicum, Beauveria bassiana, Metarhizium anisopliae, Metarhizium robertsii, Trichoderma asperelloides, Trichoderma harzianum, Trichoderma asperellum, Trichoderma viride.*

The present invention further provides a method for preparing a seed coating composition comprising:
(i) preparing a first premix comprising:
   at least one antifreeze agent in an amount ranging from about 1% to about 20% (w/w), and
   at least one thickener agent in an amount ranging from about 0.1% to about 5% (w/w);
(ii) preparing a second premix comprising:
   water in an amount ranging from about 5 to about 20% (w/w);
   at least one antifoaming agent in an amount ranging from about 0.1 to about 10% (w/w);
   at least one dispersing agent in an amount ranging from about 5 to about 20% (w/w);
   at least one biocide in an amount ranging from about 0.1 to about 10% (w/w);
   at least one agriculturally important microorganism obtained by means of solid or liquid fermentation in an amount ranging from about 10 to about 60% (w/w);
   at least one adhesive ingredient in an amount ranging from about 5 to about 20% (w/w); and
   optionally at least one colorant in an amount ranging from about 5 to about 30% (w/w);
      and
   mixing the first premix with the second premix to obtain the final formulation.

As further preferred embodiments of the present invention, the mixing of the first premix with the second premix is carried out in stainless steel containers under constant agitation of about 40 Hz to about 60 Hz, until the the suspension is homogeneous.

In a preferred method, the mixing of the first premix with the second premix is carried out for between about 30 and about 180 minutes.

The compositions, methods, and coated seeds according to the present invention may be applied to seeds of any crop plant species. Preferably, the seed is a seed of a plant selected from the group consisting of *Brachiaria* spp., *Panicum* spp., *Sorghum* spp., *Pennisetum americanum, Glycine max, Zea mays, Gossypium hirsutum, Triticum aestivum, Solanum lycopersicum, Allium cepa, Lactuca sativa, Daucus carota, Capsicum annuum, Eucalyptus* spp., *Tectona grandis, Hevea brasiliensis, Pinus* sp., *Nicotiana tabacum, Crotalaria* spp., *Helianthus annuus* and *Arachis hypogaea.*

Optionally, a composition according to the present invention includes the combination of agriculturally important microorganisms in combination with one or more chemical agents such as chemical pesticides, fungicides, insecticides and/or nematicides.

Furthermore, the composition allows the formulation to be applied at low use rates and enables the incorporation of pigments of different colors, making its use in combination with treatment packages technically and economically feasible.

The composition is intended to be used as a flowable concentrate for seed treatment (FS) formulation. However, other formulations containing these microorganisms, such as emulsions and dispersions, can also be used.

Any colorant commonly used in the art can be used in a composition according to the present invention, such as, but not limited to, red, green, blue, purple, black, pink, orange, and the like. This versatility allows the formulation to be used in conjunction with different chemical treatments, depending on its original color.

Additional embodiments and aspects of the present invention may be understood by a person skilled in the art based on their knowledge of the prior art.

The present invention discloses that a formulation composition for industrial seed treatment is capable of increasing product shelf life and improving the survival of microorganisms on chemically treated seeds. Furthermore, the agronomic effects observed in seeds treated with the Industrial Seed Treatment (IST) formulation are superior to those observed **in seeds treated with a formulation that does not comprise the composition of the present invention.**

Plant-parasitic nematodes targeted for control by a composition according to the present invention include, but are not limited to, *Pratylenchus brachyurus, Pratylenchus zea,* Pratylenchus coffea, *Meloidogyne incognita, Meloidogyne javanica, Meloidogyne exígua, Heterodera glycines, Rotylenchulus reniformis* and *Helicotilechus dihystera.*

Plant diseases that may be controlled by a composition according to the present invention include, but are not limited to *Rhizoctonia solani, Fusarium solani, Fusarium oxysporum, Fusarium graminearum, Colletotrichum gloeosporioides, Colletotrichum truncatum, Colletotrichum lindemuthianum, Macrophomina phaseolina, Sclerotinia sclerotiorum, Botrytis cinerea* and *Penicillium digitatum.*

The present invention may be used for the treatment of seeds of, but not limited to, *Brachiaria* spp. (*Brachiaria brizantha, Brachiaria decumbens, Brachiaria humidicola, Brachiaria mutica, Brachiaria ruziziensis, Brachiaria arrecta, Brachiaria dictyneura*), Panicum spp. (*Panicum maximum*), Sorghum spp., *Pennisetum americanum, Glycine max, Zea mays, Gossypium hirsutum, Triticum aestivum,* hortaliças (*Solanum lycopersicum, Allium cepa, Lactuca sativa, Daucus carota, Capsicum annuum*), florestais (Eucalyptus spp., *Tectona grandis, Hevea brasiliensis,* Pinus sp.), *Nicotiana tabacum,* ornamentais, Crotalaria spp. (*Crotalaria juncea, Crotalaria spectabilis, Crotalaria ochroleuca, Crotalaria paulina,* and *Crotalaria breviflora.*

The compositions obtained according to the present invention are liquid formulations characterized as flowable concentrates for seed treatment (FS).

The present invention discloses that applying the product in reduced slurry volumes (e.g., about 2.5 mL/kg of seed) efficiently promotes plant growth in agriculturally relevant crops, such as maize and soybean, as will be appreciated by those skilled in the art.

### EXAMPLES

### Example 1 - Physicochemical stability of the formulation

Objective - TO obtain optimal ratios of active to inert ingredients, providing adequate stability to the formulation.

Materials and Methods - A composition comprising 40% *Bacillus licheniformis,* 20% adhesive compound, 13% colorant, 8% dispersing agents, 5% antifreeze agent, 0.6% antifoaming agents, biocides and thickening agents, in addition to 13.4% water was formulated to assess its physicochemical stability.

Experimental Design - The prepared formulation was stored for 14 days under different temperature conditions (5, 28, and 54 °C) and assessed for its physical characteristics, such as appearance, color, odor, uniformity, among others, as well as chemical characteristics, which must remain within the specified limits for integrity of the chemical structure, content of active ingredients, and other relevant parameters. It is worth noting that this storage condition (54 °C for 14 days) simulates the equivalent of two years of storage at room temperature.

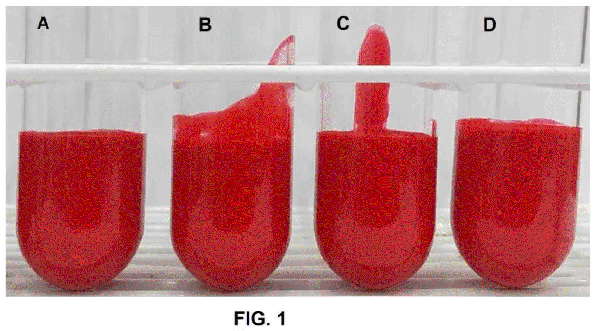

Results - Following the storage period, all physical and chemical parameters evaluated were found to retain good stability of the formulation at all tested temperatures when compared with the freshly prepared formulation, as shown in Figure 1.

### Example 2 - Biological stability of the formulation.

Objective - To assess survival of the microorganism (active ingredient) within the formulation.

Materials and Methods - A composition comprising 40% *Bacillus licheniformis,* 20% adhesive compound, 13% colorant, 8% dispersing agents, 5% antifreeze agent, 0.6% antifoaming agents, biocides and thickening agents, in addition to 13.4% water was formulated to assess the bacterial survival within the formulation.

Experimental Design - The prepared formulation was stored for 14 days under different temperature conditions (5, 28, and 54 °C) and assessed for microorganism survival. It is worth noting that this storage condition (54 °C for 14 days) simulates the equivalent of two years of storage at room temperature.

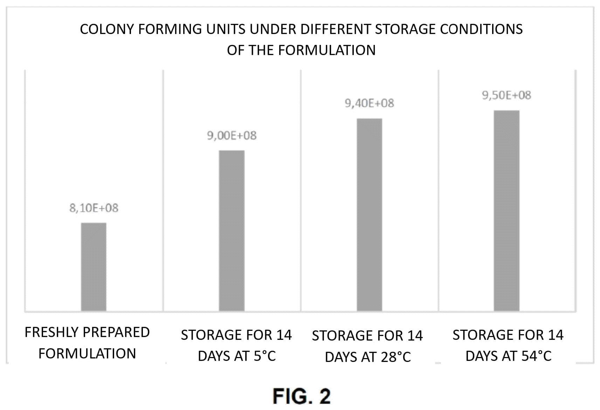

Results - After the storage period, the number of colony-forming units (CFU) remained stable at all tested temperatures when compared with the freshly prepared formulation, maintaining a bacterial concentration on the order of 10⁸ CFU/mL, as shown in Figure 2.

### Example 3 - Seed coating and adhesion.

Objective - To assess the coating and adhesion capacity of the composition of present invention on soybean and maize seeds.

Materials and Methods - A composition comprising 40% *Bacillus licheniformis,* 20% adhesive compound, 13% colorant, 8% dispersing agents, 5% antifreeze agent, 0.6% antifoaming agents, biocides and thickening agents, and 13.4% water was formulated and assessed for seed-coating performance at different application rates, as well as for the adhesion of the coating on the seeds.

The coating was evaluated based on the visual appearance of the treated seeds, and adhesion was assessed based on pigment release onto filter paper after agitation of the treated seeds in water for 10 minutes.

Experimental Design - Application rates ranging from 1.0 to 3.0 mL of formulation per kilogram of seed were evaluated in combination with the doses recommended by the manufacturers for each chemical seed treatment package assessed. The seeds were treated using a machine that simulates the same process performed by industrial machines.

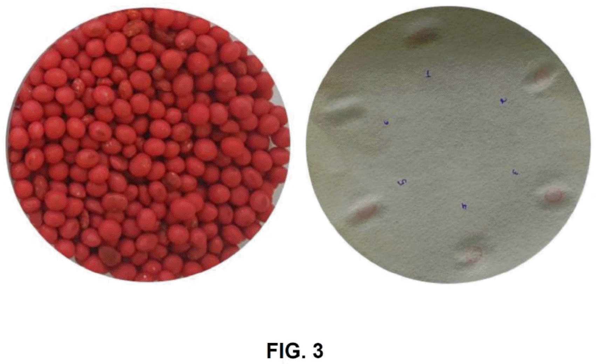

Results - An application rate of 2.5 mL of formulation per kilogram of seed was found to be optimal for providing a satisfactory visual appearance of the treated seeds, without significant release of pigment during the adhesion test, as illustrated in Figure 3.

### Example 4 - Recovery of the microorganism from treated seeds

Objective - To evaluate *Bacillus licheniformis* survival on seeds treated with the formulation of the present invention over time during storage at room temperature.

Materials and Methods - A composition comprising 40% *Bacillus licheniformis,* 20% adhesive compound, 13% colorant, 8% dispersing agents, 5% antifreeze agent, 0.6% antifoaming agents, biocides and thickening agents, and 13.4% water was used to treat soybean seeds according to the formulation application rate described in Example 3 above. Microorganism survival was evaluated every 30 days by solid plating method.

Experimental Design - Treated seeds were stored at room temperature and the counts of colony-forming units (CFU) present on the seeds were determined in triplicate every 30 days by plating on solid culture medium. Differences in survival rates were compared between seeds treated only with the active ingredient of the formulation (with no inert ingredients), seeds treated only with the formulation of the present invention, and seeds treated with the formulation of the present invention in combination with a chemical seed treatment package.

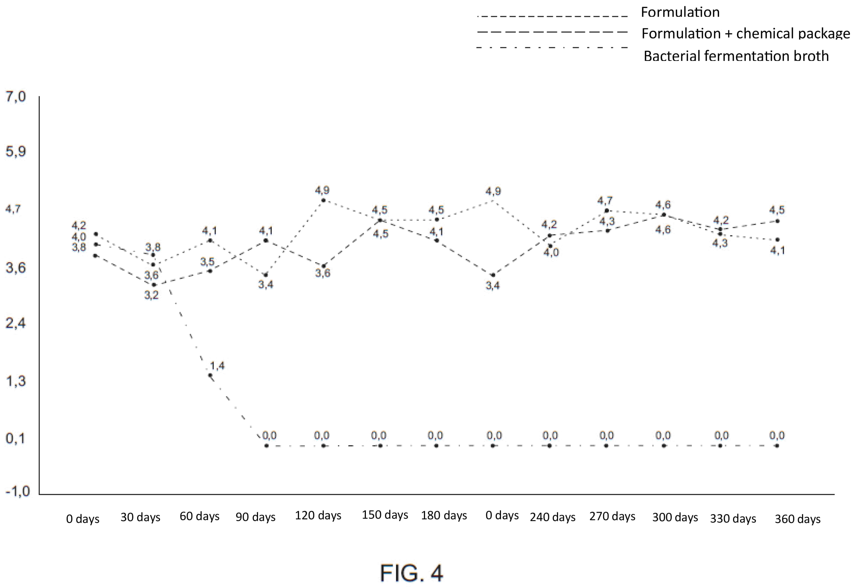

Results - In treatments comprising the bacterium formulated according to the present invention, the microorganism survived throughout the storage period assessed (360 days), as illustrated in Figure 4. The observed survival was independent of the presence of associated chemical treatment, showing that the formulation protects the bacterial cells even when chemical and biological seed treatments are used in combination. In contrast, when the seeds were treated with the bacterial fermentation broth alone, all microorganisms on the seeds were dead 90 days after treatment.

### Example 5 - Different pigments in seed coating

Objective - To assess the coating quality and adhesion performance of different pigments in the formulation.

Materials and Methods - A composition comprising 40% *Bacillus licheniformis,* 20% adhesive compound, 13% different colorants, 8% dispersing agents, 5% antifreeze agent, 0.6% antifoaming agents, biocides and thickening agents, and 13.4% water was formulated and assessed for seed-coating performance at different application rates. The coating was evaluated based on the visual appearance of the treated seeds.

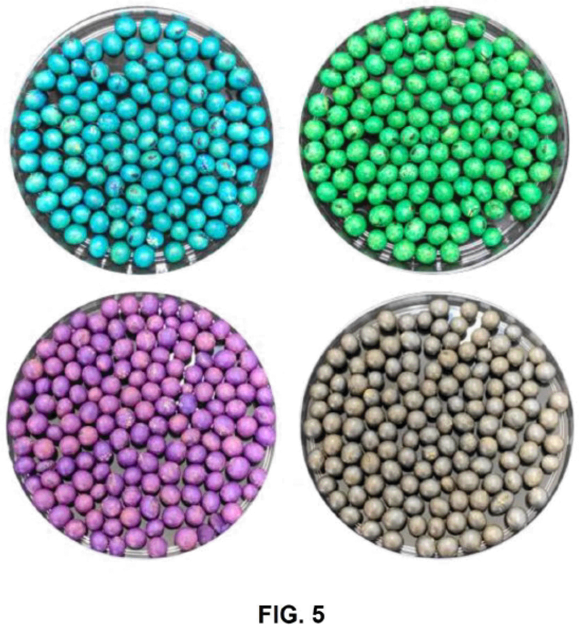

Results - The application dose of 2.5 mL of formulation, as used in Example 3, was found to optimal to provide effective seed coating when used with pigments of various colors, including blue, green, purple, and black, as evidenced in Figure 5.

Example 6 - Formulation efficacy in a field trial Objective - To evaluate the effect under field conditions of maize seeds treated with the biological formulation in terms of promoting plant growth.

Experimental Design - A completely randomized block design was employed, comprising four treatments and four replicates. Plants were arranged in four rows, each of 4 m in length and spaced 50 cm apart, resulting in a useful plot area of 16 m².

Treatments and mode of application - The maize seeds used in the field trial were previously treated using a Momesso Arktos L-2K seed treatment machine. Treatments consisted of: (1) untreated control, (2) the formulation of the present invention, (3) the chemical insecticides and fungicides, Cruiser 350 FS and Maxim XL, and (4) the combination of chemical and biological treatments of the formulation of the present invention.

The seeds were sown in the planting furrow and within the relevant post-sowing period for each assessment, the plants were evaluated for: emergence, vigor, leaf area, root length and weight, shoots length and weight, and yield.

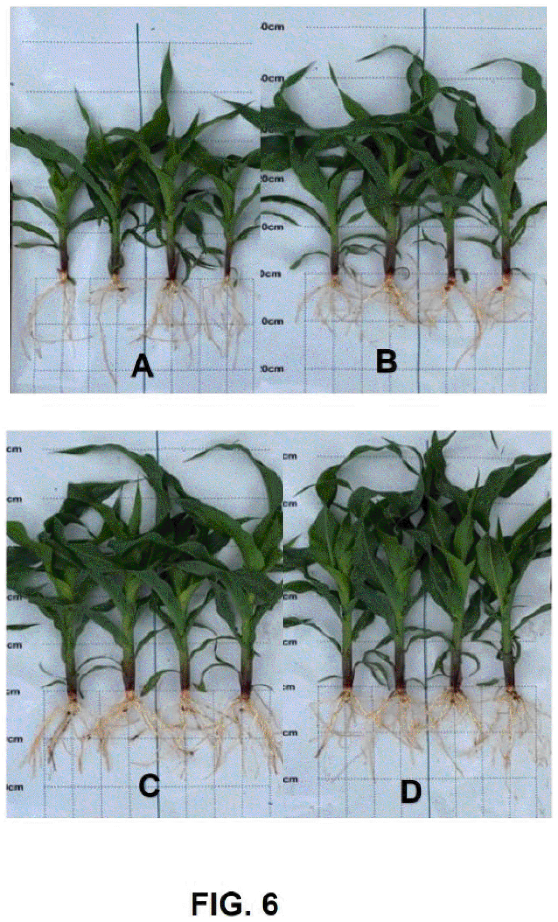

Results - Figure 6 and Table 1 show the results of the treatments. The coating of seeds with a composition of the present invention containing agriculturally important microorganisms outperformed all other treatments, from the crop establishment to yield. The biological formulation combined with the chemical treatment package (Cruiser-Maxim) showed excellent synergism, increasing yield by 9.5%, vigor by 13.2%, and leaf area by 9.2%, compared to the chemical treatment package used alone.

**TABLE 1 Biometric evaluations performed in a field trial with maize. Seeds were coated according to treatments (1)-(4) and the different responses of the association between the biological formulation of the present invention and a market standard chemical treatment were evaluated.**

| Treatment | Products | Dose | Emergence | Vigor | Leaf Area (%) | Shoot length (cm) | Root length (cm) | Shoot weight (g) | Root weight (g) | Yield (60 Kg bags/ha) |
|---|---|---|---|---|---|---|---|---|---|---|
| 1 | Control | | 2.0 | 5.0 | 59.3 | 53.7 | 15.7 | 42.2 | 12.5 | 103.0 |
| 2 | Formulation | 2.5 mL/kg | 2.2 | 7.3 | 63.2 | 63.2 | 18.3 | 63.6 | 17.5 | 117.8 |
| 3 | Cruiser | 4.8 mL/ha | 2.1 | 6.8 | 62.8 | 54.4 | 16.8 | 45.2 | 12.9 | 121.9 |
| | Maxim XL | 2 mL/kg | | | | | | | | |
| 4 | Cruiser | 4.8 mL/ha | 2.2 | 7.7 | 68.6 | 66.2 | 18.9 | 69.2 | 20.5 | 133.5 |
| | Maxim XL | 2 mL/kg | | | | | | | | |
| | Formulation | 2.5 mL/kg | | | | | | | | |

Based on the results obtained, it can be demonstrated that seed treatment with the biological formulation of the present invention can be recommended in the integrated management of various crops, for various purposes of controlling and promoting plant growth. Using *Bacillus* spp. Added to the coating components of industrially treated seeds proves to be a viable alternative for agricultural management. Furthermore, in addition to the several mechanisms of action of these specific microorganisms, this biological formulation can be expanded to include other microorganisms with numerous control pathways.

### NON-PATENT LITERATURE

BASHAN, Y., BASHAN, L.E., PRABHU, S.R. Advances in plant growth-promoting bacterial inoculant technology: formulations and practical perspectives (1998-2013). Plant Soil 378, 1-33, 2014. Doi: 10.1007/s11104-013-1956-x
BIRD, D.M., OPPERMAN, C.H., DAVIES, K.G. Interactions between bactéria and plant parasitic nematodes: now and then. Int. J. Parasitol. 33:1269-127, 2003.
CHATTERJEE A., SINGH S., AGRAWAL C., YADAV S., RAI R., RAI, L. C. Papel das algas como biofertilizante, Algal Green Chemistry: Recent Progress in Biotechnology . Ed. Rastogi RP, Pandey A., Madamwar D. Amsterdam, The Netherlands Elsevier, 189-200, 2017.
FERREIRA, A., BASTOS, R.V., MARQUES, S.C., ACIÉN-FERNANDEZ, F.G., GOUVEIA, L. Algaeculture for agriculture: from past to future. Frontiers in Agronomy, v5, 2023. Doi:10.3389/fagro.2023.1064041.
FLORENCIO, C., BORTOLETTO-SANTOS, R., FAVARO, C., GOVONI, M.B., VELLOSO, C., KLAIC, R., RIBEIRO, C., FARINAS, C., MATTTOSO, L. Avanços na produgão e formulação de inoculantes microbianos visando uma agricultura mais sustentável. Química Nova. 45. 2022. Doi: 10.21577/0100-4042.20170909.
GOULART, A. C. P., NUNES, J. C. S. Evolução e cenário atual do tratamento de sementes de soja com fungicidas no Brasil. Revista cultivar, 2021.
HENNING, A. A. Tratamento industrial de sementes mais prático e eficiente. Revista Campo & Negócio, no. 115, 2012.
HERMANN, L., LESUEUR, D. Challenges of formulation and quality of biofertilizers for successful inoculation. Appl Microbiol Biotechnol 97, 8859-8873, 2013. Doi:10.1007/s00253-013-5228-8
LUDWIG, M.P., OLIVEIRA, S., AVELAR, S.A.G., ROSA, M.P., FILHO, O.A. L., CRIZEL, R. L. Storage of Treated Soybeans and Their Effect on Seedling Performance. Tecnologia e Ciência agropecuária, v9, no.1, pp.51-56, 2015.
MACHADO, J.C. Tratamento de sementes no controle de doenças. Lavras, LAPS/FAEPE, 138 pp., 2000.
MENTEN, J. O., MORAES, M. H. D. Avanços no Tratamento e revestimento de sementes. Tratamento de sementes: Históricos, Tipos, Características e Benefício. Informativo Abrates, v.20, no.3, 2010.
PARISI, J. J. D., MEDINA, P. F. Tratamento de sementes. Instituto agronômico de Campinas (IAC) - Centro de Pesquisa e Desenvolvimento de Fitossanidade, 2013.
SAMMAURIA, R., KUMAWAT, S., KUMAWAT, P. Microbial inoculants: potential tool for sustainability of agricultural production systems. Arch Microbiol 202, 677-693, 2020. Doi:10.1007/s00203-019-01795-w
SOUMARE, A., BOUBEKRI, K., LYAMLOULI, K., HAFIDI, M., OUHDOUCH, Y., KOUISNI, L. From Isolation of Phosphate Solubilizing Microbes to Their Formulation and use as Biofertilizers: Status and Needs. Front. Bioeng. Biotechnol. 7:425, 2020. Doi:10.3389/fbioe.2019.00425
ZUCCHI T.D., MORAES L.A.B., MELO I.S. Streptomyces sp. ASBV-1 reduces aflatoxin accumulation by Aspergillus parasiticus in peanut grain. J. Appl. Microbiol. 105:2153 2160, 2008.

## Claims

1. Seed coating composition comprising:
at least one antifreeze agent in an amount ranging from about 1% to about 20% (w/w);
at least one thickener agent in an amount ranging from about 0.1% to about 5% (w/w);
water in an amount ranging from about 5 to about 20% (w/w);
at least one antifoaming agent in an amount ranging from about 0.1 to about 10% (w/w);
at least one dispersing agent in an amount ranging from about 5 to about 20% (w/w);
at least one biocide in an amount ranging from 0.1 to 10% (w/w);
at least one agriculturally useful microorganism in an amount ranging from about 10 to about 60% (w/w); and
at least one adhesive in an amount ranging from about 5 to about 20% (w/w).

2. The composition according to claim 1, wherein the composition further comprises a colorant in an amount ranging from about 5 to about 30% (w/w).

3. The composition according to claim 1, wherein the antifreeze agent is propylene glycol, glycerol, hexylene glycol, monoethylene glycol or diethylene glycol.

4. The composition according to claim 1, wherein the thickener is xanthan gum, guar gum, gum arabic or carboxymethylcellulose.

5. The composition according to claim 1, wherein the antifoaming agent is emulsified silicone.

6. The composition according to claim 1, wherein the dispersant is triestyrylphenol phosphate ether, sodium N-methyl oleyl taurate, styrene acrylic polymer, sulfosuccinate, triestyrylphenol ethoxylate and triestyrylphenol ethoxylate sulfate or polysorbate.

7. The composition according to claim 1, wherein the biocide is 1,2-benzisothiazol-3(2H)-one, potassium sorbate, methylparaben, sodium benzoate or benzyl alcohol.

8. The composition according to claim 1, wherein the adhesive is an acrylic polymer, urethane methacrylate, methacrylate ester, acrylic epoxy or acrylate.

9. The composition according to claim 1, wherein the agriculturally important microorganism is from a *genus* selected from the group consisting of *Bacillus subtilis, Bacillus amyloliquefaciens, Bacillus velezensis, Bacillus pumilus, Bacillus thuringiensis, Bacillus circulans, Bacillus haynesii, Bacillus aryabhattai, Bacillus licheniformis, Paenibacillus durus, Brevibacillus laterosporus, Pseudomonas fluorences, Pseudomoas chlororaphis, Pseudomonas protegens, Azospirillum brasilense, Bradyrhizobium japonicum, Beauveria bassiana, Metarhizium anisopliae, Metarhizium robertsii, Trichoderma asperelloides, Trichoderma harzianum, Trichoderma asperellum, Trichoderma viride.*

10. A method for preparing a seed coating composition comprising:
(i) preparing a first premix comprising:
at least one antifreeze agent in an amount ranging from about 1% to about 20% (w/w), and
at least one thickener agent in an amount ranging from about 0.1% to about 5% (w/w);
(ii) preparing a second premix comprising:
water in an amount ranging from about 5 to about 20% (w/w);
at least one antifoaming agent in an amount ranging from about 0.1 to about 10% (w/w);
at least one dispersing agent in an amount ranging from about 5 to about 20% (w/w);
at least one biocide in an amount ranging from about 0.1 to about 10% (w/w);
at least one agriculturally useful microorganism in an amount ranging from about 10 to about 60% (w/w);
at least one adhesive ingredient in an amount ranging from about 5 to about 20% (w/w); and
optionally at least one colorant in an amount ranging from about 5 to about 30% (w/w);
and
mixing the first premix with the second premix to obtain the final formulation.

11. The method according to claim 10, wherein the mixing of the first premix with the second premix is carried out in stainless steel containers, under constant agitation of about 40 Hz to about 60 Hz, until the suspension is homogeneous.

12. The method according to claim 11, wherein the mixing of the first premix with the second premix is carried out for between about 30 and about 180 minutes.

13. A method for producing coated seeds, which comprises:
mixing seeds with a composition as defined in claim 1; and homogenizing the mixture.

14. A seed coated with a composition as defined in claim 1.

15. The method according to claim 13, or the seed according to claim 14, wherein the seed is a seed from a plant selected from the group consisting of: *Brachiaria* spp., *Panicum* spp., *Sorghum* spp., *Pennisetum americanum, Glycine max, Zea mays, Gossypium hirsutum, Triticum aestivum, Solanum lycopersicum, Allium cepa, Lactuca sativa, Daucus carota, Capsicum annuum, Eucalyptus* spp., *Tectona grandis, Hevea brasiliensis, Pinus* sp., *Nicotiana tabacum, Crotalaria* spp., *Helianthus annuus, Arachis hypogaea.*
